# EUROPEAN PATENT APPLICATION

(11) **EP 3 031 409 A2**
(43) Date of publication of application: **15.06.2016**
(21) Application number: 15195735.4
(22) Date of filing: 20.11.2015
(51) Int. Cl.: A61B 17/12, A61M 25/00

(54) **ANEURYSM FILLER DELIVERY SYSTEM AND CATHETER TIP TO AID FILAMENT DELIVERY**

(30) Priority: 20.11.2014 US 201462082363 P
(71) Applicant: Cook Medical Technologies LLC, Bloomington, IN 47404 (US)
(72) Inventor: FUREY, Aidan, 2500 Valby (DK); HANSEN, Palle Munk, 4632 Bjaeverskov (DK)
(74) Representative: Maitland, Matthew

(57) **Abstract**

An improved aneurysm filler delivery system including a catheter and method of using the same is described. The catheter has at least one anchoring element at its distal tip region which is used to secure the catheter to a pre-implanted medical device, such as a stent, during the filling of an aneurysm. Such action prevents a buildup of pressure or of material from forcing the distal tip of the catheter to be forced from the aneurysm before the procedure is complete.

## Description

### FIELD OF INVENTION

The present invention relates generally to medical devices. More specifically, the invention relates to an aneurysm filler delivery system including a catheter and a tip for such a catheter which assists in keeping a distal end of a catheter stationary or relatively immobile while delivering a filling agent, such as a small intestinal submucosa (SIS) filament.

### BACKGROUND

Aneurysms are characterized by swelling in the wall of a vessel which generally results in weakness in the vessel wall. If untreated, aneurysms may continue expanding until they burst thereby causing hemorrhaging. If, for example, an aneurysm is present within an artery of the brain, and the aneurysm should burst with resulting cranial hemorrhaging, death could occur. Aneurysms result from many different causes; however, most aneurysms are caused as a result of a degenerative disease which damages the muscular coating of a vessel with resulting congenital deficiency in the muscular wall of the vessel.

One method for method for treating aneurysms is that of using a microcatheter for placing multiple very small embolic coils within the aneurysm with the expectation that fibrous material will become entrapped in the embolic coils to thereby stabilize the coils within the aneurysm. With this technique, it is possible to fill the aneurysm with embolic coils, thereby sealing off the walls of the aneurysm from the pressure of blood which exist in the adjacent vessel. Further, an implant may be placed across the aneurysm and embolic coils may be passed through the structure of the implant and into the aneurysm. The structure serves to hold the embolic coils within the stent until such time as these coils become stabilized by fibrous material growing into the coils.

Some methods of filling an aneurysm include the use of balloon catheters, which in some cases can entirely occlude the vessel to be treated and stop blood flow. In many instances, the coils that are used are made of platinum, a relatively expensive metal which results in a non-degradable, permanent implant.

There is a need for improved fillers for aneurysms and methods of delivering a biocompatible filler while having a minimal impact on the ordinary circulatory function of the patient to be treated.

### BRIEF SUMMARY

Aspects of the invention are set out in the appended claims.

In one arrangement within the following disclosure, an aneurysm filler delivery system, including a catheter is described. The catheter (which may be a microcatheter) comprises a body extending from a proximal end to a distal portion, the distal portion having a distal end. The body has a lumen formed therethrough between the proximal end and the distal end and through which a longitudinal axis is defined. The distal portion is configured to anchor itself within an aneurysm while delivering a filling agent to the aneurysm. For example, the distal portion may be configured to anchor itself on a stent that has been placed across the length of the aneurysm.

The distal portion may therefore have at least one at least one anchoring element is formed thereon. A plurality of such anchoring elements may be provided, which may, for example be spaced around the circumference of the catheter. The spacing may be substantially equal. The anchoring elements may be aligned with respect to the longitudinal axis; for example, they may be provided in at least one circumferentially-spaced array. The anchoring elements may be provided in two (or more) circumferentially-spaced arrays. In other constructions, at least some of the anchoring elements may be spaced from one another with respect to the longitudinal axis. The anchoring elements may be shaped such that, when they engage with the framework of a stent, they provide significantly greater resistance to proximal movement (e.g. the distal portion of the catheter moving through the framework, towards the lumen of the stent) than to distal movement (e.g. the distal portion of catheter moving through the framework, away from the lumen of the stent). They may be shaped such that they substantially avoid catching on the framework of a stent, when the distal portion is introduced into the aneurysm, for instance moving distally through the framework of the stent, away from the lumen of the stent. Conversely, they may generally catch on the framework of the stent, when forced proximally through the framework of the stent, out of the aneurysm and towards the lumen of the stent.

The or each anchoring element may extend at least radially from the body of the catheter. The or each anchoring element may also (or instead) extend generally towards the proximal end, from the distal end.

The at least one anchoring element may include at least one hook and/or at least one flap. Such a hook may bend toward the proximal end of the catheter. Such a flap may be formed from a portion of the catheter body; it may extend radially outward and from the distal end toward the proximal end of the catheter.

The at least one anchoring element may be sufficiently resilient to retain the distal portion in an anchored position within the aneurysm while delivering a filling agent to the aneurysm. It may also be sufficiently flexible to allow the user to move the distal portion proximally, out of the aneurysm. For example, it may be sufficiently flexible to avoid fracturing during such movement. It may deform elastically during such movement (though in some cases it could instead deform plastically).

The distal portion of the catheter may be formed with a number of bends therein that assist in anchoring the distal portion within the aneurysm. The catheter may be formed such that it bends back on itself a plurality of times (for instance, in a substantially unstressed state) so as to define a convoluted portion having a width perpendicular to said longitudinal axis, this width being sufficient to anchor the distal portion within the aneurysm. The convoluted portion may have a regular shape, such as a spiral, or it may have an irregular shape. The width might be, for instance, greater than 0.25mm, greater than 0.5mm or greater than 1 mm, depending for example on the size of the apertures in the framework of the stent through which it should pass. The bends may be relatively abrupt. For example, each bend may have a radius of curvature that is of the same order of magnitude as the radius of the catheter at that point. Each bend may be, for example, between 60 and 120 degrees; it may be approximately 90 degrees. So that the distal portion may fit within an aneurysm, the straight-line distance from the proximal end of the convoluted portion to the distal end of the catheter may be selected based on, for instance, the size of the aneurysm being treated. This distance may be less than 15mm (e.g. for a giant aneurysm), less than 10 mm (e.g. for a large aneurysm), less than 5mm, less than 2.5mm (e.g. for a small aneurysm). The convoluted portion may be sufficiently resilient to retain the distal portion in an anchored position within the aneurysm while delivering a filling agent to the aneurysm. It may also be sufficiently flexible to allow the user to move the distal portion proximally, out of the aneurysm. It may deform elastically during such movement. For this reason, or otherwise, the distal portion may comprise a shape-memory material, such as a shape-memory alloy. The convoluted portion may deform elastically (for instance, from its substantially unstressed configuration) when a straightening member is passed through the distal portion, thereby straightening the bends (at least partially) in the convoluted portion.

The catheter may generally have an outer diameter in the range of about 1 to about 9 French. More specifically, it may have an outer diameter in the range of about 1 to about 4 French. The catheter may generally have a length in the range of about 75cm to about 175cm and more specifically in the range of about 100cm to about 150cm.

The aneurysm filler delivery system may further include a receptacle for a filling agent. This receptacle is typically in communication with the catheter and may, for example, be directly connected to the catheter. The filling agent may include at least one filament, such as a filament derived from a biological material, and more specifically, a filament derived from extracellular matrix or small intestine submucosa filament. Such filaments may be wound around a spool provided within the receptacle. The catheter and/or the receptacle may be in communication with a fluid port, through which a fluid (e.g. saline) may be introduced that forces the filament out of the end port of the catheter, into the aneurysm.

In another arrangement within the following disclosure, there is described an aneurysm filler delivery system, including a catheter. The catheter (which may be a mircocatheter) comprises a body extending from a proximal end to a distal portion, the distal portion having a distal end. The body has a lumen formed therethrough between the proximal end and the distal end and through which a longitudinal axis is defined. The distal portion has at least one slit formed from an outer surface to the lumen.

The at least one slit may extend distally to the distal end. Alternatively, it may stop short of the distal end; it may be spaced apart from the distal end.

A plurality of such slits may be provided, which may, for example be spaced around the circumference of the catheter. The spacing may be substantially equal. The slits may be aligned with respect to the longitudinal axis; for example, they may be provided in a circumferentially-spaced array.

In a more general arrangement within the following disclosure, a medical device comprising a catheter is described. The catheter comprises a body extending from a proximal end to a distal portion, the distal portion having a distal end. The body has a lumen formed therethrough between the proximal end and the distal end. A longitudinal axis is defined through the lumen. The distal portion comprises at least one anchoring element formed thereon.

In another general arrangement within the following disclosure, a catheter is described. The catheter comprises a body extending from a proximal end to a distal portion, the distal portion having a distal end, the body having a lumen formed therethrough between the proximal end and the distal end and through which a longitudinal axis is defined. The distal portion has at least one slit formed from an outer surface to the lumen.

In another arrangement within the following disclosure, a method of deploying a catheter and filling an aneurysm is described. The method comprises the steps of providing a medical implant near a neck region of the aneurysm; providing a catheter, a body extending from a proximal end to a distal portion, the distal portion having a distal end, the body having a lumen formed therethrough between the proximal end and the distal end, the distal portion comprising at least one anchoring element formed thereon; introducing the catheter to the patient's body; positioning the exit port within the aneurysm; engaging the medical implant with the anchoring element; filling the aneurysm with a filling agent; and removing the catheter from the patient.

Further objects, features, and advantages of the present invention will become apparent from consideration of the following description and the appended claims when taken in connection with the accompanying drawings.

### BRIEF DESCRIPTION OF THE DRAWINGS

FIG. 1A-1C are perspective views of a prior art catheter filling an aneurysm;
FIG. 2A is a perspective view of an inventive aneurysm filler delivery system including a catheter with anchoring members at its distal tip filling an aneurysm in accordance with the principles of the present invention;
FIG. 2B is a close-up view of the anchoring members and distal tip of the catheter of FIG. 2A;
FIG. 2C-2E are close-up view (side view, head-on view, and perspective views, respectively) of the distal tip of FIG. 2B;
FIG. 2F is a perspective view of a modification to the distal tip of FIG. 2A for a catheter of an aneurysm filler delivery system according to a further embodiment of the present invention;
FIG. 3A is a perspective view of another distal tip of a catheter of an aneurysm filler delivery system in accordance with another embodiment of the present invention;
FIG. 3B is a perspective view of a modification to the distal tip of FIG. 3A in accordance with a further embodiment of the present invention;
FIG. 3C is a close-up view of a distal end of a catheter of an aneurysm filler delivery system in accordance with a further embodiment of the present invention;
FIG. 3D-3E are schematic views of the catheter depicted in FIG. 3C;
FIG. 4A is a perspective view of an inventive catheter with a slitted distal end according to one embodiment of the present invention; and
FIG. 4B is a perspective view of an inventive catheter with a different slit configuration at its distal end in accordance with another embodiment of the present invention.

### DETAILED DESCRIPTION OF THE INVENTION

The description that follows is not intended to limit the scope of the invention in any manner, but rather serves to enable those skilled in the art to make and use the invention.

It is to be understood that the figures are schematic and do not show the various components to their actual scale. In many instances, the figures show scaled up components to assist the reader.

In this description, when referring to a device, a delivery system, a catheter, or a medical implant, the term distal is used to refer to an end of a component which in use is furthest from the physician during the medical procedure, including within a patient. The term proximal is used to refer to an end of a component closest to the physician and in practice in or adjacent an external manipulation part of the deployment or treatment apparatus.

The terms "substantially" or "about" used herein with reference to a quantity includes variations in the recited quantity that are equivalent to the quantity recited, such as an amount that is equivalent to the quantity recited for an intended purpose or function. In the case of a numerical quantity, the terms "substantially" or "about" shall mean a range consisting of a value 50% less than the recited value to a value 50% greater than the recited value, inclusive.

It is to be understood that any type of catheter is meant when the term "catheter" is used in this disclosure, inclusive of microcatheters.

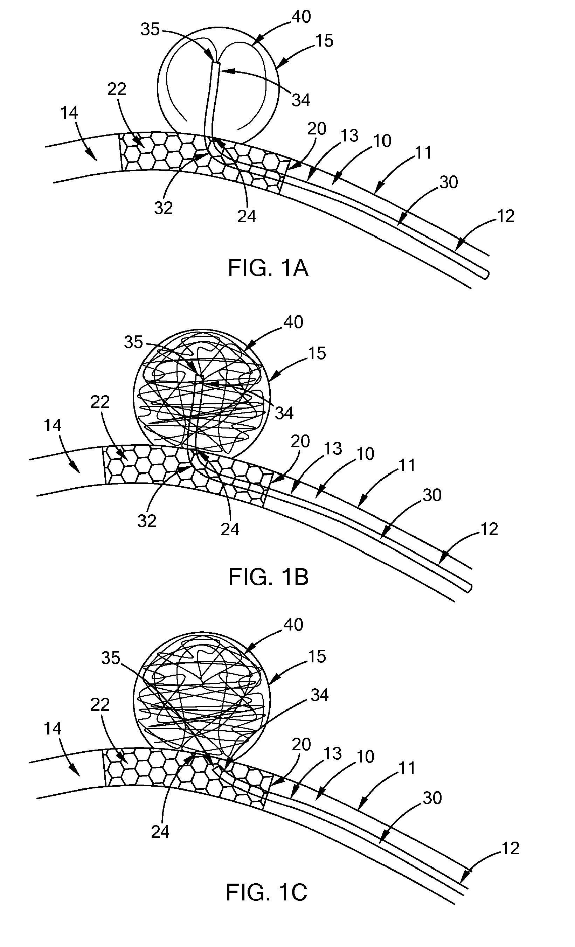

FIG. 1A-1C is a sequential diagrammatic representation of one problem to be solved by an inventive aneurysm filler delivery system comprising a catheter. In FIG. 1A, aneurysm 15 is formed off of the vessel wall 11 of blood vessel 10. Medical implant (or stent) 20 has been placed across the length of the aneurysm 15, creating a portion of the lumen 13 of the blood vessel 10 which is distal to the aneurysm (distal vessel portion 14) and a portion which is proximal to it (proximal vessel portion 12.)

The stent 20 has a stent structure made of interconnected struts and connecting segments. There are empty spaces 22 within the framework of the stent, and in particular a catheter 30 can be passed through a catheter space 24 to enter the aneurysm. In doing this, the catheter 30 forms bend 32, which in FIG. 1A is depicted as being about 90 degrees.

The catheter has a proximal end closest to the practitioner and a distal end farthest from the practitioner, and a catheter lumen is defined therethrough and extending from the proximal end to the distal end. Further, a longitudinal axis is defined within the tubular-shaped catheter.

The catheter has a distal tip 34 at the distal end of the catheter. This distal tip 34 can either be unitarily formed with the remainder of the catheter body, or it can be a separate part which is attached to the catheter body by any conventional means, reversibly or irreversibly. The distal tip 34, at its extreme distal end, is where exit port 35 is formed.

In the embodiment of FIG. 1A, a fiber or filamentary material is being used to fill the aneurysm. The filamentary material could be of any type deemed suitable for performing a medical function and could be a polysaccharide, a biocompatible polymeric thread, or other biocompatible material. Specific examples include but are not limited to: woven polyester (such as DACRON®); polyamide (such as Nylon); expanded polytetrafluoroethylene (ePTFE, GORE-TEX®); and bioremodelable materials such as extracellular matrix (ECM) material, such as submucosa, renal capsule membrane, dermal collagen, dura mater, pericardium, fascia lata, serosa, peritoneum, and basement membrane layers. Examples of submucosa include intestinal submucosa, stomach submucosa, urinary bladder submucosa, and uterine submucosa.

In one embodiment, this fiber may be made of a biological material. In a specific embodiment, the biological material may be derived from extracellular matrix (ECM) or small intestinal submucosa (SIS). The biological material is formed into a fiber and provided on a bobbin or spool within a handle portion of the aneurysm filler delivery system. The spool may have just one fiber wound around it, or may hold two fibers, or any other number of fibers depending on the needs of the application.

FIG. 1A illustrates a point in time at which fiber 40 has just been forced from the catheter body through exit port 35. A receptacle containing a substantial amount of the fibrous material may be part of the system. Such a receptacle can, for instance, be substantially spherical, and may therefore contain a substantially spherical spool of fibrous material, free to rotate in any direction, within it. Ejecting the material can be done by providing a pressurized liquid, such as saline solution, through the catheter 30 in order to force the fiber 40 out of the catheter and into the aneurysm 15. In another embodiment, rather than a spooled SIS fragment or other biological material, the filler is a series of platinum coils which are loaded consecutively in the catheter.

Further progression of this process is shown in FIG. 1 B. At this point, most of the filler is out of the catheter, and the aneurysm 15 is becoming quite full. Thus, the procedure has nearly concluded.

However, a problem emerges and the result is shown in FIG. 1C. When the aneurysm is nearly full, spatial constraints and/or pressure cause the catheter distal tip 34 to be dislodged and in some cases completely removed from the aneurysm. This can lead to the delivery of filler outside of the aneurysm, underfilling of the aneurysm, and other issues.

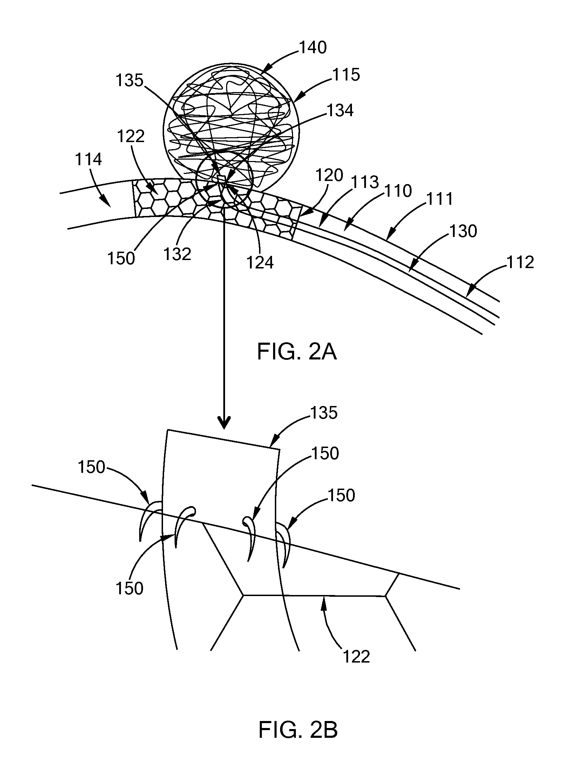

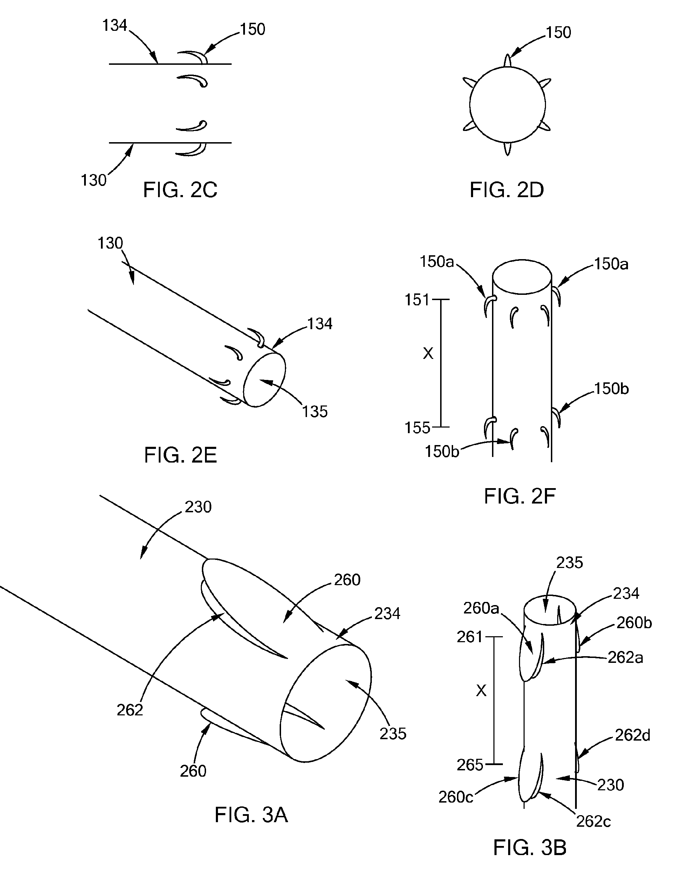

To solve this problem, the catheter 130 of the aneurysm filler delivery system of FIG. 2A has been given hooks or prongs 150. In one embodiment, there is one hook 150 present at the distal tip 134 of the catheter 130. In another embodiment, there are at least two hooks 150 circumferentially spaced at any convenient interval around the distal tip 134. When the prongs 150 engage the stent 120, the added pressure and special constraints as the aneurysm 115 fills with fiber 140 does not cause the catheter 130 to dislodge; rather, the hooks 150 catch on the stent and the catheter stays in place.

FIG. 2B is a close up view of the hooks 150. These hooks jut out from the catheter body and in the depicted embodiment, point back in the proximal direction. The hooks may be made of any material - a plastic, a rubber, a shape memory metal, or the like - so long as it is strong enough to withstand the additional pressure imparted by the filling process. In one embodiment, the prongs 150 are made of a resilient but flexible material, such that a pushing force generated by the filling of the aneurysm keeps the hooks 150 in their original shape, but a stronger pulling force in the proximal direction, such as by the hand of the practitioner conducting the procedure, causes the hooks 150 to start to bend distally such that the catheter can be retracted from the body of the patient.

In one embodiment, the catheter may be formulated comprising polyurethane as a structural binder (i.e. resin) ingredient, for example polyether-polyurethane or polyester-polyurethane. The plastic formulation that makes up the remainder of the catheter can comprise a structural binder ingredient that is sealingly compatible with polyurethane, or if the tip is other than polyurethane, then the structural binder ingredient can be sealingly compatible with any material which constitutes the structural binder of the distal tip. Generally, the structural binders of the distal tip and the remainder of the catheter comprise the same, or similar, formulations for the best sealing compatibility; i.e. when the distal tip comprises polyurethane, the remainder of the catheter also comprises polyurethane.

The distal tip and the remainder of the catheter body may comprise polyurethane, but other inert plastic materials besides polyurethane may be used to manufacture catheters, for example, polyethylene, poly(ethylene terephthalate) and other polyesters, polypropylene, polyamides such as nylon, and the like.

Additionally, radiopaque elements can be used in portions of the catheter to aid in visualization of the process for which the catheter is employed. Radiopaque elements can be used solely toward the distal tip region, or can be placed at intervals through the length of the catheter.

FIG. 2C-2E illustrate an embodiment in which the anchoring elements are represented by six hooks. The hooks are oriented such that they point back in the proximal direction. The six hooks are spaced substantially evenly around the circumference of the catheter body, with approximately 60 degrees separating the anchoring elements. They lie adjacent the exit port 135 formed at the end of the distal tip 134.

Turning now to FIG. 2F, a modification to the distal tip of the catheter of FIG. 2A-2E is illustrated. In this embodiment, the catheter has two sets of anchoring members, in this case hooks, placed around its exterior at its distal tip region 134. The first set of hooks 150a is more distal than the second set of hooks 150b. The first set of hooks 150a creates a first set of implant contact points at a first location 151 near the distal end of the catheter. The second set of hooks 150b creates a second set of implant contact points at a second location 155 proximal to the distal end of the catheter. The length between first location 151 and second location 155 is represented as a length X. Such a configuration can allow for variable placement of the catheter for improved filling and adjustment depending on the depth of the aneurysm. In another aspect, the multiple sets of hooks 150a and 150b can allow for a stronger grip on the implant by providing multiple points of contact on the structure of the implanted device.

In another embodiment, the catheter has three sets of anchoring members at its distal tip region in a similar configuration to those shown in FIG. 2F. In a further embodiment, the catheter has four sets of anchoring members arranged circumferentially around the distal tip region of the catheter at different distances from the distal end. Further embodiments can include five sets of anchoring members, six sets of anchoring members, or more than six sets of anchoring members.

Another embodiment of the present invention is shown in FIG. 3A. In this embodiment, the anchoring elements of catheter 230 are represented by flared flaps 260. The flaps 260 lie adjacent the exit port 235 of the catheter 230 at its distal tip 234. The flaps can be cut from the wall of the catheter 230 and biased to flare away from the catheter body when unconstrained. The flaps create a space 262 which is in fluid communication with the lumen of the vessel.

In the embodiment of FIG. 3A, the flaps can catch on the strut structure of a medical implant at an aneurysm, such as a stent, or they can serve to simply increases the diameter of the device when it has passed through an opening in the implant. However, the flaps 260 must be pliant enough that a practitioner can still effect removal of the catheter 230 while providing a force in the proximal direction. The flaps 260, also allow for pressure release, as excess saline solution or other loading fluid can be at least in part expelled via spaces 262.

The depicted embodiment of FIG. 3A only shows a cylindrical catheter with two flaps, but other configurations can include more, such as 3, 4, 5, 6, 8, or more flaps 262.

Turning now to FIG. 3B, a modification to the distal tip of the catheter of FIG. 3A is illustrated. In this embodiment, the catheter has two sets of flaps cut or formed around its exterior at its distal tip region 234. The first set of flaps, comprising flaps 260a and 260b, is more distal than the second set of flaps, comprising flaps 260c and 260d. The first set of flaps 260a/260b creates a first set of implant contact points within spaces 262a and 262b at a first location 261 near the distal end of the catheter. The second set of flaps 260c/260d creates a second set of implant contact points within spaces 262c and 262d at a second location 265 proximal to the distal end of the catheter. The length between first location 261 and second location 265 is represented as a length X. Such a configuration can allow for variable placement of the catheter for improved filling and adjustment depending on the depth of the aneurysm. In another aspect, the multiple sets of flaps 260a/260b and 260c/260d can allow for a stronger grip on the implant by providing multiple points of contact in spaces 262a/262b/262c/262d on the structure of the implanted device.

In another aspect, the number of flaps cut or formed in one set, circumferentially, in the distal tip region 234 of the catheter 230 can be three. In another aspect, the number can be four. There can also be five, six, eight, or more flaps in such arrangements. The number of flaps 260a does not have to be equal to the number of flaps 260b, nor do their positions circumferentially around the catheter have to align.

In another embodiment, the catheter has three sets of flaps at its distal tip region in a similar configuration to those shown in FIG. 3B. In a further embodiment, the catheter has four sets of flaps arranged circumferentially around the distal tip region of the catheter at different distances from the distal end. Further embodiments can include five sets of flaps, six sets of flaps, or more than six sets of flaps.

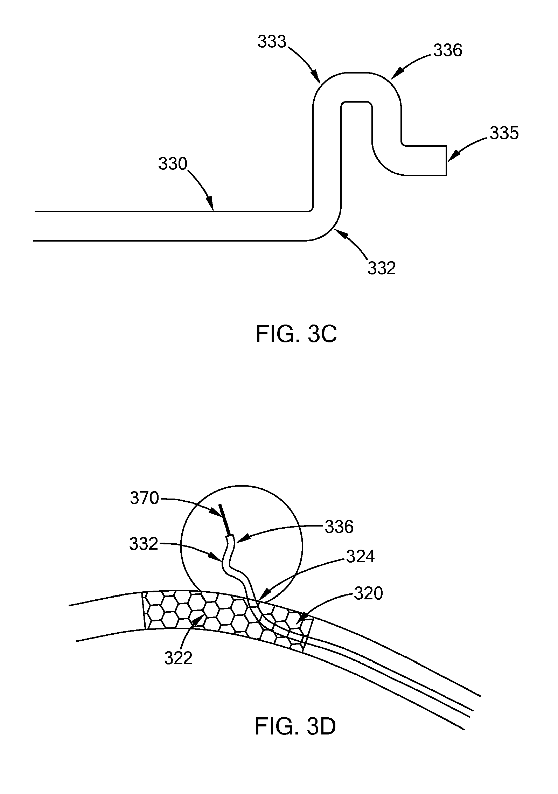

FIG. 3C-3E illustrate another embodiment of the device of the present disclosure. Here, a number of bends are formed in the distal tip 334 of the catheter 330. The catheter 330 has a proximal end, then a first bend 332 of about 90 degrees. Following the catheter body distally, second bend 333 is formed distal to first bend 332, and is also about 90 degrees. Distal to second bend 333 is third bend 336, which is also about 90 degrees, and fourth bend 338 which is a final bend of about 90 degrees. As may be seen, the catheter bends back on itself and thus defines a convoluted portion, including bends 332, 333 and 336, that has a width perpendicular to the longitudinal axis of the catheter 330 and that assists in anchoring the distal tip 334 within the aneurysm.

The angles of these bends need not be 90 degrees; any configuration of bends in the distal tip 334 of a catheter 330 which will allow it to be locked into position when deployed in an aneurysm for filler delivery will suffice. In one embodiment, the distal tip 334 is made of a shape memory material that can be straightened but maintains a bent configuration as illustrated.

The catheter tip may comprise at least one shape memory material. Shape memory materials reversibly transform between a lower temperature phase (martensite) and a higher temperature phase (austenite) while passing through a transition temperature therebetween. Shape memory alloys have the desirable property of becoming rigid when heated above the transition temperature.

A shape memory alloy suitable for the present invention is an alloy comprising nickel and titanium. When a nickel-titanium shape memory alloy is heated above the transition temperature, the material undergoes a phase transformation from martensite to austenite, such that the material starts with a substantial amount of elasticity but at the transition temperature begins to become rigid. The transition temperature is dependent on the relative proportions of the alloying elements nickel (Ni) and titanium (Ti) and the optional inclusion of alloying additives. Often the proportions of Ni and Ti are selected so that the material is austenite at body temperature.

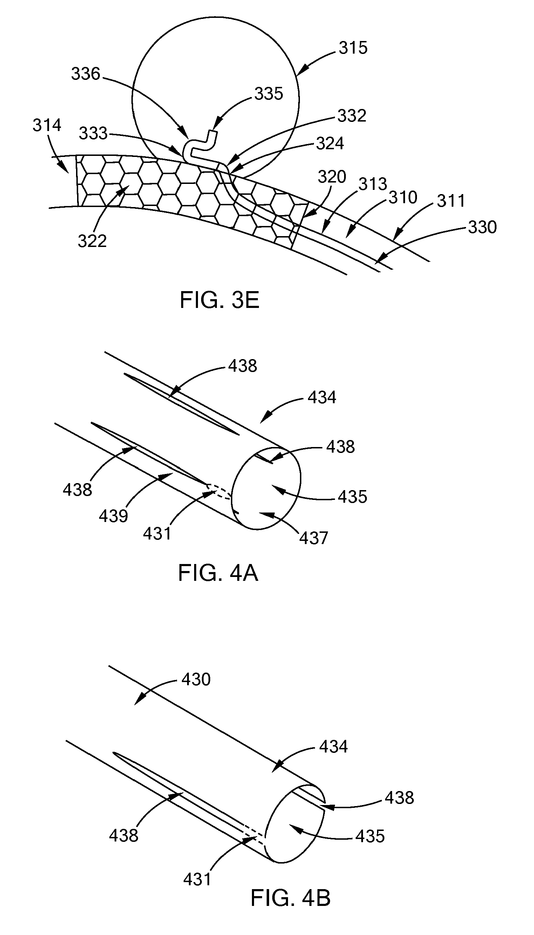

To deploy the catheter 330 through the structure of the medical implant or stent 320, in one embodiment the distal tip 334 is straightened. FIG. 3D shows straightening with a straightening member 370, with convoluted portion deforming elastically. In one embodiment, the straightening member 370 is a wire guide. In another embodiment, the straightening member 370 is an inner dilator. FIG. 3E shows the tip reverting to its heat-set shape after retraction of the straightening member 370. This allows the distal tip region to define the convoluted portion shown in FIG. 3C, which has a width perpendicular to the longitudinal axis that is larger than the apertures of the stent. In this way, the distal tip catches within the aneurysm and resists the pressure as the aneurysm fills.

In another embodiment, the convoluted portion has a more regular shape, such as in a spiral. In another embodiment, a pigtail catheter configuration is employed.

FIG. 4A shows yet another embodiment of the present invention. Because one problem to be addressed by the anchoring element is resistance to pressure, a solution to this problem can reduction of pressure rather than adaptation to it. In this instance, slits 438 having slit boundaries 439 are cut or formed through the walls of the catheter 430. These slits allow fluid to flow through so that additional pressure from fluids (such as excess saline flushing solution) in the aneurysm are allowed to escape the aneurysm as it fills. The slits 438 formed in distal tip 434 as illustrated in FIG. 4A do not extend all the way to the distal end of the catheter body and thus do not have fluid connections with exit port 435, although they do have fluid connections with lumen 437. This is shown by tip space 431, surrounded by a dotted boundary, being of a solid construction. However, in another embodiment, such as the embodiment of FIG. 4B, the slits 438 do continue all the way to the distal end. Such a variant may help to realize a higher release of fluid pressure due to greater flexibility at the distal end of the catheter body.

In another embodiment, the inventive catheter tip may be formed as its own separate element and then attached to the distal end of any conventional catheter body. Thus, a catheter having an inventive tip structure may either be formed monolithically; have its anchoring elements (such as hooks) formed separately and attached to the distal end of a standard catheter; or a tip portion having a lumen and the anchoring elements formed thereon may be attached as one part, reversibly or irreversibly, to a separate catheter body.

The catheters described above may be utilized in a variety of methods to fill an aneurysm. Such a method may comprise a number of steps, the first of which is providing a medical implant (such as a stent with a strut structure) near a neck region of the aneurysm; providing a catheter as described throughout this disclosure and comprising at least one anchoring element formed thereon; introducing the catheter to the patient's body; positioning the exit port within the aneurysm; engaging the medical implant with the anchoring element (for example, in the case where the anchoring element is a hook, catching said hook on the wire struts of the stent already present in the body of the patient); filling the aneurysm with a filling agent, such as SIS fiber or platinum microcoils; and removing the catheter from the patient.

A person having skill in the art will readily appreciate that the method will be adjusted based on the particular embodiment of the tip of the catheter. For instance, when a catheter having a slit rather than a hook is used, the method will not include a step of engaging the medical implant with the anchoring element; rather, the catheter will simply be passed into the aneurysm and fill it without such an engagement step.

While the apparatus of the invention has been described above with reference to certain specific embodiments thereof, it is to be clearly understood that these embodiments have been given for purposes of illustration only and are not intended to be limiting. The scope of the invention is bounded only by the scope of the claims which are set out hereafter.

### CLAUSES

1. A catheter comprising:
   a body extending from a proximal end to a distal portion, the distal portion having a distal end, the body having a lumen formed therethrough between the proximal end and the distal end and through which a longitudinal axis is defined, the distal portion comprising at least one anchoring element formed thereon.
2. The catheter of clause 2 wherein the at least one anchoring element comprises at least one hook, the at least one hook bending toward the proximal end of the catheter.
3. The catheter of clause 3 wherein the at least one hook is made of a flexible material.
4. The catheter of clause 1 wherein the at least one anchoring element comprises a flap, the flap being formed from a portion of the catheter body, the flap extending outward from the distal end toward the proximal end of the catheter.
5. The catheter of clause 1 wherein the at least one anchoring element is formed as one or more bends in the distal portion of the catheter.
6. The catheter of clause 5 wherein the distal portion has a spiral shape.
7. The catheter of clause 5 wherein the distal portion has a first configuration comprising at least one bend of at least approximately 90 degrees relative to the longitudinal axis.
8. The catheter according to clause 1 wherein the distal portion is formed integrally with the body of the catheter.
9. The catheter according to clause 1 wherein the distal portion is formed as a separate part from the body of the catheter and is attached to the catheter body by an attachment mechanism.
10. A catheter comprising:
   a body extending from a proximal end to a distal portion, the distal portion having a distal end, the body having a lumen formed therethrough between the proximal end and the distal end and through which a longitudinal axis is defined;
   the distal portion having at least one slit formed from an outer surface to the lumen.
11. The catheter according to clause 10 wherein the at least one slit extends distally to the distal end.
12. A method of treating an aneurysm in a body vessel in which a medical implant has been placed adjacent a neck region of an aneurysm, the method comprising:
   providing a catheter comprising a body extending from a proximal end to a distal portion, the distal portion having a distal end, the body having a lumen formed therethrough between the proximal end and the distal end, the distal portion comprising at least one anchoring element formed thereon;
   positioning the exit port within the aneurysm;
   anchoring the at least one anchoring element on the medical implant; and
   delivering a filling agent to the aneurysm through the exit port of the catheter.
13. The method according to clause 12 wherein the at least one anchoring element comprises at least one hook, the hook bending toward the proximal end of the catheter, the at least one hook being made of a flexible material.
14. The method according to clause 12 wherein the at least one anchoring element comprises a flap, the flap being formed from a portion of the catheter body, the flap extending outward from the distal end toward the proximal end of the catheter.
15. The method according to clause 12 wherein the at least one anchoring element comprises a first configuration wherein the tip region of the catheter has one or more bends.
16. The method according to clause 15 wherein the first configuration comprises a spiral shape.
17. The method according to clause 15 wherein the first configuration comprises at least one bend of at least approximately 90 degrees.
18. The method according to clause 15 further comprising a second configuration wherein a wire guide is passed through the lumen of the catheter to substantially straighten the tip region.
19. The method according to clause 12 wherein the filling agent is a filament derived from a biological material.
20. The method according to clause 19 wherein the filling agent comprises at least one small intestine submucosa filament.

## Claims

1. An aneurysm filler delivery system, including a catheter, preferably a microcatheter, the catheter comprising:
a body extending from a proximal end to a distal portion, the distal portion having a distal end, the body having a lumen formed therethrough between the proximal end and the distal end and through which a longitudinal axis is defined, the distal portion being configured to anchor itself within an aneurysm while delivering a filling agent to the aneurysm.

2. The system of Claim 1, wherein at least one at least one anchoring element is formed on said distal portion, preferably wherein a plurality of said anchoring elements are provided, more preferably wherein said plurality of anchoring elements are spaced around the circumference of the catheter, still more preferably provided in at least one circumferentially-spaced array and optionally in two or more circumferentially-spaced arrays.

3. The system of claim 2 wherein the at least one anchoring element comprises at least one hook, preferably wherein the at least one hook bends toward the proximal end of the catheter.

4. The system of claim 2, wherein the at least one anchoring element comprises at least one flap, the at least one flap being formed from a portion of the catheter body, preferably wherein the at least one flap extends radially outward and from the distal end toward the proximal end of the catheter.

5. The system of any one of claims 2 to 4, wherein the at least one anchoring element is sufficiently resilient to retain the distal portion in an anchored position within the aneurysm while delivering a filling agent to the aneurysm; and
wherein the at least one anchoring element is sufficiently flexible to allow the user to move the distal portion proximally, out of the aneurysm.

6. The system of claim 1 wherein, in the distal portion of the catheter is formed with a number of bends therein that assist in anchoring the distal portion within the aneurysm, preferably wherein the catheter is formed such that it bends back on itself one or more times so as to define a convoluted portion having a width perpendicular to said longitudinal axis, said width being sufficient to anchor the distal portion within the aneurysm.

7. The catheter of claim 6, wherein the distal portion has a spiral shape.

8. The system of any preceding claim, wherein the distal portion is formed integrally with the body of the catheter.

9. The system of any one of claims 1 to 7, wherein the distal portion is formed as a separate part from the body of the catheter and is attached to the catheter body by an attachment mechanism.

10. An aneurysm filler delivery system, including a catheter, preferably a microcatheter, the catheter comprising:
a body extending from a proximal end to a distal portion, the distal portion having a distal end, the body having a lumen formed therethrough between the proximal end and the distal end and through which a longitudinal axis is defined;
the distal portion having at least one slit formed from an outer surface to the lumen.

11. The system of Claim , wherein a plurality of said slits are provided, preferably wherein said slits are spaced around the circumference of the catheter, more preferably wherein said slits are provided in a circumferentially-spaced array.

12. The system of Claim 10 or Claim 11, wherein the at least one slit is elongate in a direction generally parallel to said longitudinal axis.

13. The system of any preceding claim, further comprising a receptacle for a filling agent, the receptacle communicating with said catheter, preferably further comprising a handle, with said receptacle being provided within said handle.

14. The system of any preceding claim, further comprising a filling agent loaded within said catheter, the filling agent comprising at least one filament, preferably filament derived from a biological material, and more preferably derived from extracellular matrix or small intestine submucosa filament.

15. The system of Claim 14, when dependent upon Claim 13, wherein the at least one filament is wound around a spool provided within said receptacle.
